Europäisches Patentamt

European Patent Office

Office européen des brevets

(19)

(11) EP 1 369 079 A1

(12) EUROPEAN PATENT APPLICATION

(43) Date of publication:
10.12.2003 Bulletin 2003/50

(51) Int Cl.$^7$: A61B 3/113, G02B 26/08

(21) Application number: 03011826.9

(22) Date of filing: 26.05.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 28.05.2002 US 156654

(71) Applicant: Alcon Inc.
6331 Hunenberg (CH)

(72) Inventors:
• Zepkin, Neil
  Oviedo Florida 32762 (US)
• Nguyen, Phouc Khanh
  Winter Springs, Florida 32708 (US)

(74) Representative: Hanna, Peter William Derek et al
Hanna, Moore & Curley,
11 Mespil Road,
Dublin 4 (IE)

(54) Zoom device for eye tracker control system and associated methods

(57) The invention provides a zooming mechanism for use in an eye tracking system (100) includes a pyramidal prism that has either a plurality of reflective facets or of transmissive facets meeting at an apex. An incident light beam (35,45) directed onto each facet of the prism is reflected/refracted onto a planar surface substantially normal to the optical axis (34,44), to form a plurality of light spots (21,22,23,24) arrayed about an optical axis. The prism is translatable along the optical axis between axial positions for altering a spacing of the light spots without substantially changing their size. Preferably the spots are directed onto a boundary defined by two adjoining surfaces of the eye (10) having different coefficients of reflection. Reflected energy from each of the plurality of positions is detected, and a size of a pattern (39,49) formed by the light spots is adjustable without substantially changing a diameter of the individual light spots.

FIG. 1

EP 1 369 079 A1

## Description

### FIELD OF THE INVENTION

The invention relates generally to eye tracking devices for ophthalmic laser surgical systems, and more particularly to such a device that has a zoom capability.

### BACKGROUND OF THE INVENTION

The use of lasers to erode a portion of a corneal surface is known in the art to perform corrective surgery. In the field of ophthalmic medicine, photorefractive keratectomy (PRK), phototherapeutic kerat ectomy (PTK), laser in situ keratomileus (LASIK), and laser epithelial keratomileusis (LASEK) are procedures for laser correction of focusing deficiencies of the eye by modification of corneal profile.

In these procedures, surgical errors due to application of the treatment laser during unwanted eye movement can degrade the refractive outcome of the surgery. The eye movement or eye positioning is critical since the treatment laser is centered on the patient's theoretical visual axis which, practically speaking, is approximately the center of the patient's pupil. However, this visual axis is difficult to determine, owing in part to residual eye movement and involuntary eye movement, known as saccadic eye movement. Saccadic eye movement is high-speed movement (i.e., of very short duration, 10-20 milliseconds, and typically up to 1° of eye rotation) inherent in human vision and is used to provide a dynamic scene to the retina. Saccadic eye movement, while being small in amplitude, varies greatly from patient to patient due to psychological effects, body chemistry, surgical lighting conditions, etc. Thus, even though a surgeon may be able to recognize some eye movement and can typically inhibit/restart a treatment laser by operation of a manual switch, the surgeon's reaction time is not fast enough to move the treatment laser in correspondence with eye movement.

A system for performing eye tracking has been described in U.S. Pat. Nos. 5,632,742; 5,752,950; 5,980,513; 6,302,879; and 6,315,773, which are commonly owned with the present application,

### SUMMARY OF THE INVENTION

It is an object of the present invention to provide an eye tracking method and system that is used in conjunction with a laser system for performing corneal correction.

Another object is to provide such a method and system that includes a zooming feature for changing a separation of light spots incident upon the eye, collectively called the probe beam.

A further object is to provide such a system and method in which use of the zooming feature does not change a size of the probe beam.

In accordance with the present invention, a zooming-mechanism for use in an eye tracking system is disclosed that, in a first embodiment, comprises a pyramidal prism having a plurality of reflective facets meeting at an apex, oriented so that the apex points along an optical axis. Means are provided for directing an incident light beam onto each facet of the prism. Each incident light beam is reflected away from the prism in a direction pointing toward the apex. The directing means is adapted to produce a plurality of reflected beams that, when incident upon a planar surface substantially normal to the optical axis, form a plurality of light spots arrayed about the optical axis.

A second embodiment of the zooming mechanism comprises a pyramidal transmissive prism that has a plurality of facets meeting at an apex, the apex pointing along an optical axis. Means are provided for directing an incident light beam onto each facet of the prism. Each incident light beam is refracted within the prism to form a refracted beam in a direction pointing toward the apex. When the plurality of refracted beams are incident upon a planar surface substantially normal to the optical axis, a plurality of light spots are formed that are arrayed about the optical axis.

In both embodiments, means are provided for translating the prism along the optical axis between a first position wherein the light spots are separated by a first spacing and a second position wherein the light spots are separated by a second spacing that is smaller than the first spacing. The light spots thereby, in a preferred embodiment, have a substantially equal size with the prism in the first and the second positions.

In a system incorporating the zoom mechanism of the present invention, a light source generates a modulated light beam, for example, in the near-infrared 905-nanometer wavelength region. An optical delivery arrangement including the zoom mechanism converts each laser modulation interval into the plurality of light spots, which are focused such that they are incident on a corresponding plurality of positions located on a boundary whose movement is coincident with that of eye movement. The boundary can be defined by two visually adjoining surfaces having different coefficients of reflection. The boundary can be a naturally occurring boundary (e.g., the iris/pupil boundary or the iris/sclera boundary) or a manmade boundary (e.g., an ink ring drawn, imprinted or placed on the eye, or a contrast-enhancing tack affixed to the eye). Energy is reflected from each of the positions located on the boundary receiving the light spots. An optical receiving arrangement detects the reflected energy from each of the positions. Changes in reflected energy at one or more of the positions is indicative of eye movement.

One aspect of the method of the present invention comprises a method for sensing eye movement. This method comprises the steps of directing a plurality of light beams onto a plurality of positions on a boundary defined by two adjoining surfaces of the eye to form a plurality of light spots. The two surfaces are selected to have different coefficients of reflection. Reflected energy from each of the plurality of positions is detected, wherein changes in the reflected energy at one or more of the positions is indicative of eye movement. In order to retain the light spots on the boundary, a size of a pattern formed by the plurality of light spots is adjusted on the plurality of positions. This adjustment, in a preferred embodiment, is performed without substantially changing a diameter of the individual light spots.

## BRIEF DESCRIPTION OF THE DRAWINGS

FIG. 1 is a block diagram of an eye movement tracking system in accordance with the present invention.
FIG. 2 is a block diagram of an optical arrangement for the focusing optics in the eye tracking system.
FIG. 3 is a block diagram of an optical arrangement for the focusing optics in the eye tracking system using a pyramidal zoom device.
FIG. 4 is a schematic diagram of a translatable reflective prism being used in a zoom mechanism in a first position.
FIG. 5 is a schematic diagram of the translatable reflective prism of FIG. 3 in a second position.
FIG. 6 is a schematic diagram of a translatable transmissive prism being used in a zoom mechanism in a first position.
FIG. 7 is a schematic diagram of the translatable transmissive prism of FIG. 5 in a second position.

## DETAILED DESCRIPTION OF THE INVENTION

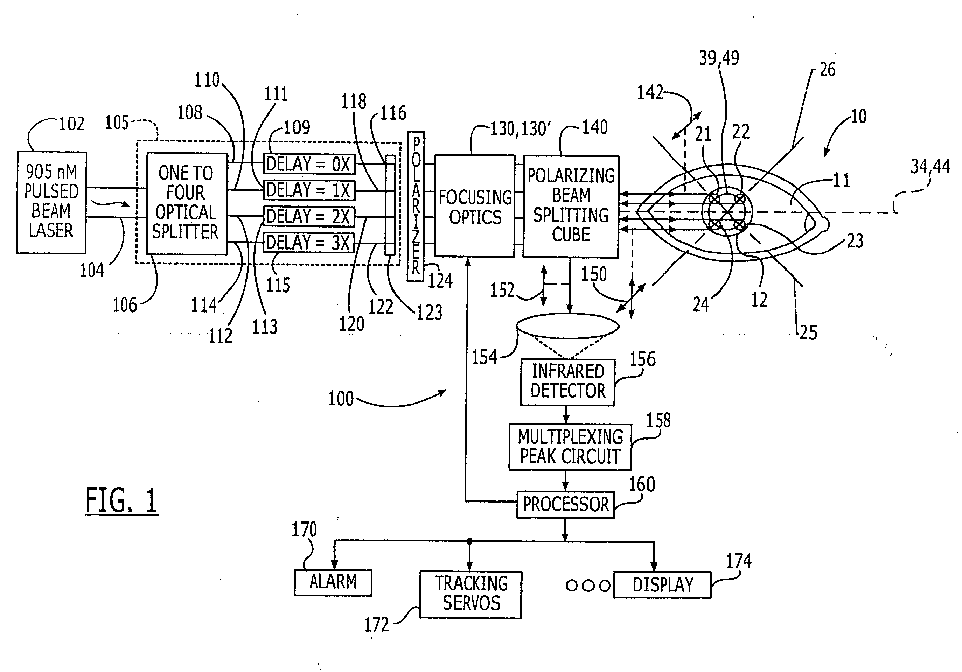

A description of a preferred embodiment of the present invention will now be presented with reference to FIGS. 1-7.

A preferred embodiment system, referenced generally by numeral 100, for carrying out the method of the present invention will now be described with the aid of the block diagram shown in FIG. 1. System 100 may be broken down into a delivery portion and a receiving portion. The delivery portion projects light spots 21, 22, 23, and 24 onto eye 10, while the receiving portion monitors reflections caused by light spots 21, 22, 23, and 24.

The delivery portion includes a laser 102 transmitting light through optical fiber 104 to an optical fiber assembly 105 that splits and delays each pulse from laser 102 into preferably four equal-energy pulses. An exemplary laser 102 comprises a 905-nanometer pulsed diode, although this is not intended as a limitation. Assembly 105 includes a one-to-four optical splitter 106 that outputs four pulses of approximately equal energy into optical fibers 108, 110, 112, 114. Such optical splitters are commercially available (e.g., model HLS2X4 manufactured by Canstar and model MMSC-0404-0850-A-H-1 manufactured by E-Tek Dynamics). In order to use a single processor to process the reflections caused by each pulse transmitted by fibers 108,110,112, and 114, each pulse is uniquely multiplexed by a respective fiber optic delay line (or optical modulator) 109, 111, 113, and 115. For example, delay line 109 causes a delay of zero, i.e., DELAY=Ox where x is the delay increment; delay line 111 causes a delay of x, i.e., DELAY=1x; etc.

The pulse repetition frequency and delay increment x are chosen so that the data rate of system 100 is greater than the speed of the movement of interest. In terms of saccadic eye movement, the data rate of system 100 must be on the order of at least several hundred hertz. For example, a system data rate of 4 kHz is achieved by (1) selecting a small but sufficient value for x to allow processor 160 to handle the data (e.g., 250 nanoseconds), and (2) selecting the time between pulses from laser 102 to be 250 microseconds (i.e., laser 102 is pulsed at a 4-kHz rate).

The four equal-energy pulses exit assembly 105 via optical fibers 116, 118, 120, and 122, which are configured as a fiber optic bundle 123. Bundle 123 arranges optical fibers 116, 118, 120, and 122 in a manner that produces a square (dotted line) with the center of each fiber at a corner thereof.

Light from assembly 105 is passed through an optical polarizer 124 that attenuates the vertical component of the light and outputs horizontally polarized light beams as indicated by arrow 126. Horizontally polarized light beams 126 pass to focusing optics 130, where the spacing between beams 126 is adjusted based on the boundary of interest. Additionally, a zoom capability can be provided to allow for adjustment of the size of the pattern formed by spots 21-24. This capability allows system 100 to adapt to different patients, boundaries, etc. In particular embodiments, the spots 21-24 are focused on a boundary between the iris and the sclera or on a boundary between the iris and the pupil.

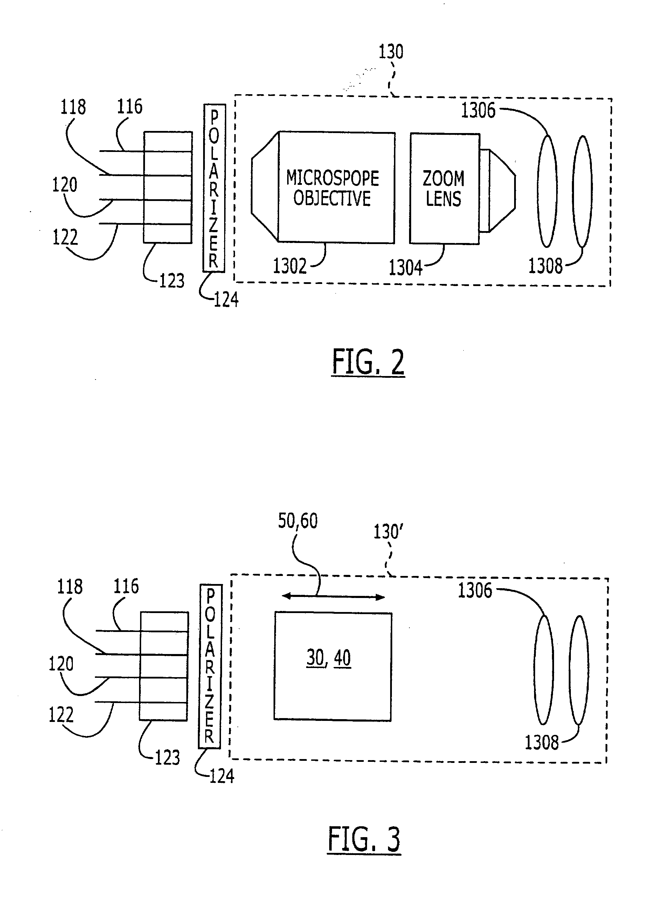

While a variety of optical arrangements are possible for focusing optics 130, one such arrangement is shown byway of example in FIG. 2. In FIG. 2, fiber optic bundle 123 is positioned at the working distance of microscope objective 1302. The numerical aperture of microscope objective 1302 is selected to be equal to the numerical aperture of fibers 116, 118, 120, and 122. Microscope objective 1302 magnifies and collimates the incoming light. Zoom lens 1304 provides an additional magnification factor for further tunability. Collimating lens 1306 has a focal length that is equal to its distance from the image of zoom lens 1304 such that its output is collimated. The focal length of imaging lens 1308 is the distance to the eye such that imaging lens 1308 focuses the light as four sharp spots on the corneal surface of the eye.

The zoom lens 1304 as described above changes the probe beam geometry, that is, the inscribed circle that contains all the probe beams, in order to accommodate varying object sizes and boundaries. A standard zoom lens **1304** may be used for this purpose, however, the dynamic range for laser tracking devices using standard zoom lenses is limited because the individual probe beam size is changed in direct proportion to the overall probe beam geometry.

In order to optimize dynamic range, the magnification of the overall probe beam geometry, that is, the inscribed circle of spots **21-24**, would preferably be decoupled from that of the individual beam size. Two embodiments of a system and method for achieving such a decoupling will now be presented with reference to FIGS. 3-7, with FIG. 3 representing a block diagram of an optical arrangement for the focusing optics **130'** in the eye tracking system using a pyramidal zoom device.

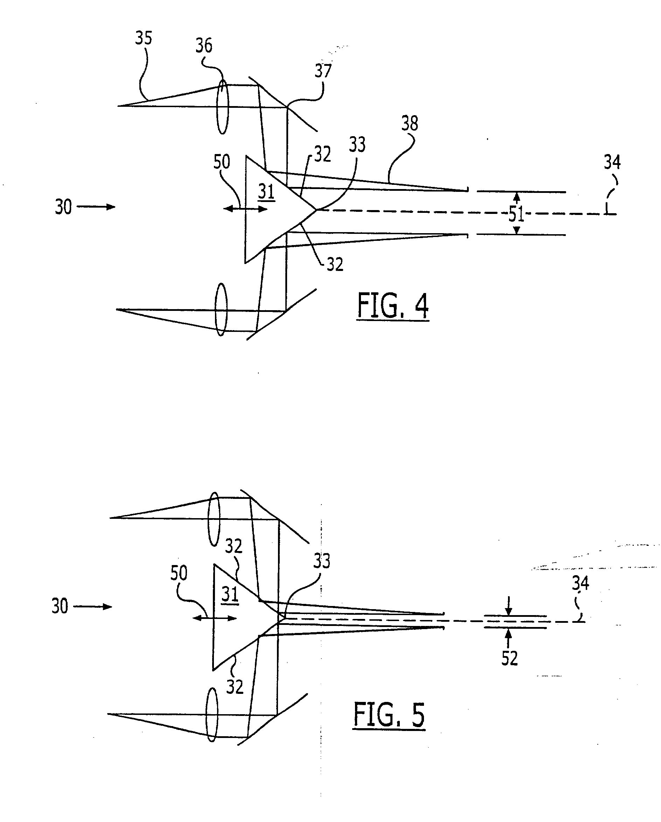

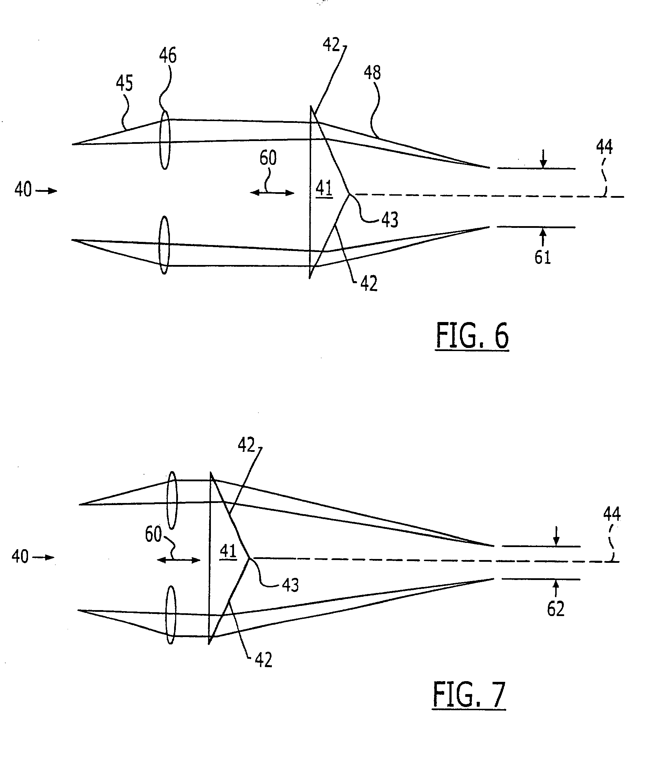

A first embodiment of the zoom mechanism **30** comprises a pyramidal prism **31** having a plurality of, in a preferred embodiment four, reflective facets **32** (FIGS. 4 and 5). It will be understood by one of skill in the art that FIGS. 4 and 5 (and subsequently discussed FIGS. 6 and 7) are highly schematic representations in two dimensions for ease of presentation, four-sided pyramidal prisms being well known in the art.

The facets **32** meet at an apex **33** that points along an optical axis **34**. It will also be understood by one of skill in the art that by "apex" is meant herein the point or sector at which the facets reach their smallest dimension, and that the prism may in fact comprise a truncated pyramid without a pointed apex.

An incident light beam **35** is directed onto each facet **32** of the prism **31** by an optical arrangement comprising a focusing lens **36** that is positioned to receive an incident light beam **35** and is adapted to image the respective incident light beam **35** to an image plane.

In a preferred embodiment a generally planar mirror **37** is disposed in the optical pathway to receive the respective incident light beam **35** downstream of the respective focusing lens **36** and to reflect the respective incident light beam **35** onto a selected prism facet **32**. Preferably the mirror **37** is oriented substantially parallel to the selected prism facet **32**. The mirror **37** is present in a preferred embodiment to serve as a "folding" mirror for reducing a size of the mechanism **30**.

Each incident light beam **35** is then reflected away from the prism **31** in a direction pointing toward the apex **33**, producing a plurality of reflected beams **38**. When the reflected beams **38** are incident upon a planar surface substantially normal to the optical axis **34** to form the plurality of light spots **21-24** (FIG. 1) arrayed substantially on an inscribed circle **39** about the optical axis **34** substantially in a square pattern.

A second embodiment of the zoom mechanism **40** comprises a pyramidal transmissive prism **41** having a plurality of, in a preferred embodiment four, facets **42** (FIGS. 6 and 7). The facets **42** meet at an apex **43** that points along an optical axis **44**.

An incident light beam **45** is directed onto each facet **42** of the prism **41** by an optical arrangement comprising a focusing lens **46** that is positioned to receive an incident light beam **45** and is adapted to image the respective incident light beam **45** to an image plane.

Each incident light beam **45** refracted within the prism **41** to form a refracted beam **48** in a direction pointing toward the apex **43**. The plurality of refracted beams **48**, when incident upon a planar surface substantially normal to the optical axis **44**, form the plurality of light spots **21-24** arrayed substantially in a square on an inscribed circle **49** (FIG. 1) about the optical axis **44.**

The zooming mechanisms **30,40** further comprise a mechanism **50,60** for translating the prism **31,41** along the optical axis **34,44** between a first position (FIGS. 4 and 6) wherein the light spots **21-24** are separated by a first spacing **51,61** and a second position (FIGS. 5 and 7) wherein the light spots **21-24** are separated by a second spacing **52,62** smaller than the first spacing **51,61**. In this arrangement, the light spots **21-24** advantageously have a substantially equal size with the prism **31,41** in the first and the second positions. The translating mechanism **50,60** may comprise, for example, a motorized translating stage such as is known in the art that is under processor **160** control.

Referring again to FIG. 1, polarizing beam splitting cube **140** receives horizontally polarized light beams **126** from focusing optics **130**. Polarization beamsplitting cubes are well known in the art. Byway of example, cube **140** is a model 10FC16PB.5 manufactured by Newport-Klinger. Cube **140** is configured to transmit only horizontal polarization and reflect vertical polarization. Accordingly, cube **140** transmits only horizontally polarized light beams **126** as indicated by arrow **142**. Thus it is only horizontally polarized light that is incident on eye **10** as spots **21-24**. Upon reflection from eye **10**, the light energy is depolarized (i.e., it has both horizontal and vertical polarization components), as indicated by crossed arrows **150**. The vertical component of the reflected light is then directed/reflected as indicated by arrow **152**. Thus cube **140** serves to separate the transmitted light energy from the reflected light energy for accurate measurement.

The vertically polarized portion of the reflection from spots **21-24** is passed through focusing lens **154** for imaging onto an infrared detector **156.** Detector **156** passes its signal to a multiplexing peak detecting circuit **158**, which is essentially a plurality of peak sample-and-hold circuits, a variety of which are well known in the art. Circuit **158** is configured to sample (and hold the peak value from) detector **156** in accordance with the pulse repetition frequency of laser **102** and the delay $x$. For example, if the pulse repetition frequency of laser **102** is 4 kHz, circuit **158** gathers reflections from spots **21-24** every 250 microseconds.

By way of example, infrared detector 156 is an avalanche photodiode model C30916E manufactured by EG&G. For a given transmitted laser pulse, the detector output will consist of four pulses separated in time by the delays associated with optical delay lines **109, 111, 113,** and **115** shown in FIG. 1. These four time-separated pulses are fed to peak-and-hold circuits. Input enabling signals are also fed to the peak-and-hold circuits in synchronism with the laser fire command. The enabling signal for each peak and hold circuit is delayed by delay circuits. The delays are set to correspond to the delays of delay lines **109, 111, 113,** and **115** to allow each of the four pulses to be input to the peak-and-hold circuits. The reflected energy associated with a group of four spots is collected as the detector signal is acquired by all four peak and hold circuits. At this point, an output multiplexer reads the value held by each peak-and-hold circuit and inputs them sequentially to processor **160**.

The values associated with the reflected energy for each group of four spots (i.e., each pulse of laser **102**) are passed to a processor **160**, where horizontal and vertical components of eye movement are determined. For example, let $R_{21}$, $R_{22}$, $R_{23}$, and $R_{24}$ represent the detected amount of reflection from one group of spots **21-24**, respectively. A quantitative amount of horizontal movement is determined directly from the normalized relationship

$$\frac{\left(R_{21} + R_{24}\right) - \left(R_{22} + R_{23}\right)}{R_{21} + R_{22} + R_{23} + R_{24}}$$

while a quantitative amount of vertical movement is determined directly from the normalized relationship

$$\frac{\left(R_{21} + R_{22}\right) - \left(R_{23} + R_{24}\right)}{R_{21} + R_{22} + R_{23} + R_{24}}$$

Note that normalizing (i.e., dividing by $R_{21} + R_{22} + R_{23} + R_{24}$) reduces the effects of variations in signal strength.

Once processed, the reflection differentials indicating eye movement (or the lack thereof) can be used in a variety of ways. For example, an excessive amount of eye movement may be used to trigger an alarm **170**. In addition, the reflection differential may be used as a feedback control for tracking servos **172** used to position an ablation laser. Still further, the reflection differentials can be displayed on display **174** for monitoring or teaching purposes.

Additionally, the detected reflected energy from light spots **21-24** may be analyzed in the processor **160** to determine a change in pupil size as determined by the reflection differentials and the spacing of the light spots **21-24**. As it is desired to retain the light spots **21-24** on a selected eye surface boundary, here coincident with the circle **39,49**, means are provided under direction of the processor **160** for directing the translating mechanism **50,60** to translate the prism **31,41** in a direction for retaining the light spots **21-24** on the selected boundary **39,49**, without substantially altering the diameters of the light spots **21-24**.

The advantages of the present invention are numerous. Eye movement is sensed in accordance with a non-intrusive method and apparatus. The present invention will find great utility in a variety of ophthalmic surgical procedures without any detrimental effects to the eye or interruption of a surgeon's view. Further, data rates needed to sense saccadic eye movement are easily and economically achieved.

Although the invention has been described relative to a specific embodiment thereof, there are numerous variations and modifications that will be readily apparent to those skilled in the art in the light of the above teachings. It is therefore to be understood that, within the scope of the appended claims, the invention may be practiced other than as specifically described.

**Claims**

1. A zooming mechanism for use in an eye tracking system comprising:

   a pyramidal prism having a plurality of reflective facets meeting at an apex, the apex pointing along an optical axis;
   means for directing an incident light beam onto each facet of the prism, each incident light beam reflected away from the prism in a direction pointing toward the apex, the directing means adapted to produce a plurality of reflected beams that, when incident upon a planar surface substantially normal to the optical axis, form a plurality of light spots arrayed about the optical axis; and
   means for translating the prism along the optical axis between a first position wherein the light spots are separated by a first spacing and a second position wherein the light spots are separated by a second spacing smaller than the first spacing.

2. The mechanism recited in Claim 1, wherein the light spots have a substantially equal size with the prism in the first and the second positions.

3. The mechanism recited in Claim 1, wherein the directing means comprises a plurality of focusing lenses, each focusing lens positioned to receive a respective one of the plurality of incident light beams and adapted to image the respective incident light beam to an image plane.

**4.** The mechanism recited in Claim 3, wherein the directing means further comprises a plurality of mirrors, each mirror disposed to receive the respective incident light beam downstream of the respective focusing lens and to reflect the respective incident light beam onto a selected prism facet.

**5.** The mechanism recited in Claim 4, wherein each mirror comprises a planar mirror that is oriented substantially parallel to the selected prism facet.

**6.** The mechanism recited in Claim 1, wherein the light spots are arrayed substantially on inscribed circle.

**7.** The mechanism recited in Claim 1, wherein the plurality of facets comprise four facets, the incident light beam comprises four light beams, and the plurality of light spots comprise four light spots arrayed substantially in a square pattern.

**8.** A zooming mechanism for use in an eye tracking system comprising:

a pyramidal transmissive prism having a plurality of facets meeting at an apex, the apex pointing along an optical axis;
means for directing an incident light beam onto each facet of the prism, each incident light beam refracted within the prism to form a refracted beam in a direction pointing toward the apex, the plurality of refracted beams, when incident upon a planar surface substantially normal to the optical axis, forming a plurality of light spots arrayed about the optical axis; and
means for translating the prism along the optical axis between a first position wherein the light spots are separated by a first spacing and a second position wherein the light spots are separated by a second spacing smaller than the first spacing.

**9.** The mechanism recited in Claim 8, wherein the light spots have a substantially equal size with the prism in the first and the second positions.

**10.** The mechanism recited in Claim 8, wherein the directing means comprises a plurality of focusing lenses, each focusing lens positioned to receive a respective one of the plurality of incident light beams and adapted to image the respective incident beam to an image plane.

**11.** The mechanism recited in Claim 8, wherein the light spots are arrayed substantially on inscribed circle.

**12.** The mechanism recited in Claim 8, wherein the plurality of facets comprise four facets, the incident light beam comprises four light beams, and the plurality of light spots comprise four light spots arrayed substantially in a square pattern.

**13.** A system for sensing eye movement comprising:

an optical delivery arrangement for directing a plurality of incident beams onto a plurality of positions on a boundary defined by two adjoining surfaces of the eye having different coefficients of reflection to form a plurality of light spots;
an optical receiving arrangement for detecting reflected energy from each of the plurality of positions, wherein changes in the reflected energy at one or more of the positions is indicative of eye movement; and
means for adjusting a size of a pattern formed by the plurality of light spots on the plurality of positions.

**14.** The system recited in Claim 13, wherein the size adjusting means are adapted to avoid substantially changing a diameter of the individual light spots when the size is adjusted.

**15.** The system recited in Claim 13, further comprising optical means for converting each pulse of a pulsed light beam into the plurality of incident beams and for forming the light spots therefrom.

**16.** The system recited in Claim 13, wherein the adjusting means comprises a zooming mechanism comprising:

a pyramidal prism having a plurality of reflective facets meeting at an apex, the apex pointing along an optical axis;
means for directing an incident light beam onto each facet of the prism, each incident light beam reflected away from the prism in a direction pointing toward the apex, the directing means adapted to produce a plurality of reflected beams that, when incident upon a planar surface substantially normal to the optical axis, form a plurality of light spots arrayed about the optical axis; and
means for translating the prism along the optical axis between a first position wherein the light spots are separated by a first spacing and a second position wherein the light spots are separated by a second spacing smaller than the first spacing.

**17.** The system recited in Claim 16, wherein the translating means are adapted to avoid substantially altering a size of the light spots with the prism in the first and the second positions.

**18.** The system recited in Claim 13, further comprising means for analyzing the detected reflected energy and for directing the translating means to translate the prism in a direction for retaining the light spots on the boundary.

**19.** The system recited in Claim 13, wherein the adjusting means comprises a zooming mechanism comprising:

> a pyramidal transmissive prism having a plurality of facets meeting at an apex, the apex pointing along an optical axis;
> means for directing an incident light beam onto each facet of the prism, each incident light beam refracted within the prism to form a refracted beam in a direction pointing toward the apex, the plurality of refracted beams, when incident upon a planar surface substantially normal to the optical axis, forming the plurality of light spots arrayed about the optical axis; and
> means for translating the prism along the optical axis between a first position wherein the light spots are separated by a first spacing and a second position wherein the light spots are separated by a second spacing smaller than the first spacing, the light spots having a substantially equal size with the prism in the first and the second positions.

**20.** The system recited in Claim 19, further comprising means for analyzing the detected reflected energy and for directing the translating means to translate the prism in a direction for retaining the light spots on the boundary.

**21.** A method for adjusting a spacing of a plurality of light spots directed onto an eye in an eye movement sensor comprising the steps of:

> directing an incident light beam onto each facet of a pyramidal prism having a plurality of reflective facets meeting at an apex, the apex pointing along an optical axis, each incident light beam reflected away from the prism in a direction pointing toward the apex, for producing a plurality of reflected beams that, when incident upon a planar surface substantially normal to the optical axis, form a plurality of light spots arrayed about the optical axis; and
> translating the prism along the optical axis between a first position wherein the light spots are separated by a first spacing and a second position wherein the light spots are separated by a second spacing smaller than the first spacing.

**22.** The method recited in Claim 21, wherein, in the translating step, the light spots have a substantially equal size with the prism in the first and the second positions.

**23.** The method recited in Claim 21, wherein the directing step comprises directing each of the plurality of incident light beams onto a respective each one of a plurality of focusing lenses, each focusing lens adapted to image the respective incident beam to an image plane.

**24.** The method recited in Claim 23, wherein the directing step further comprises disposing a mirror downstream of each focusing lens to reflect the respective incident light beam onto a selected prism facet.

**25.** The method recited in Claim 24, wherein each mirror comprises a planar mirror that is oriented substantially parallel to the selected prism facet.

**26.** The method recited in Claim 21, wherein the light spots are ar rayed substantially on inscribed circle.

**27.** The method recited in Claim 21, wherein the plurality of facets comprise four facets, the incident light beam comprises four light beams, and the plurality of light spots comprise four light spots arrayed substantially in a square pattern.

**28.** A method for adjusting a spacing of a plurality of light spots directed onto an eye in an eye movement sensor comprising the steps of:

> directing an incident light beam onto each facet of a pyramidal transmissive prism having a plurality of reflective facets meeting at an apex, the apex pointing along an optical axis, each incident light beam refracted within the prism to form a refracted beam in a direction pointing toward the apex, the plurality of refracted beams, when incident upon a planar surface substantially normal to the optical axis, forming a plurality of light spots arrayed about the optical axis; and
> translating the prism along the optical axis between a first position wherein the light spots are separated by a first spacing and a second position wherein the light spots are separated by a second spacing smaller than the first spacing.

**29.** The method recited in Claim 28, wherein, in the translating step, the light spots have a substantially equal size with the prism in the first and the second positions.

**30.** The method recited in Claim 28, wherein the directing step comprises directing each of the plurality of incident light beams onto a respective each one of a plurality of focusing lenses, each focusing lens adapted to image the respective incident beam to an image plane.

31. The method recited in Claim 28, wherein the light spots are arrayed substantially on inscribed circle.

32. The method recited in Claim 28, wherein the plurality of facets comprise four facets, the incident light beam comprises four light beams, and the plurality of light spots comprise four light spots arrayed substantially in a square pattern.

33. A method for sensing eye movement comprising the steps of:

directing a plurality of light beams onto a plurality of positions on a boundary defined by two adjoining surfaces of the eye having different coefficients of reflection to form a plurality of light spots;
detecting reflected energy from each of the plurality of positions, wherein changes in the reflected energy at one or more of the positions is indicative of eye movement; and
adjusting a size of a pattern formed by the plurality of light spots on the plurality of positions.

34. The method recited in Claim 33, wherein the size adjusting step is performed without substantially changing a diameter of the individual light spots.

35. The method recited in Claim 33, further comprising converting each pulse of a pulsed light beam into the plurality of light beams for forming the light spots therefrom.

36. The method recited in Claim 33, wherein the adjusting step comprises the steps of:

directing an incident light beam onto each facet of a pyramidal prism having a plurality of reflective facets meeting at an apex, the apex pointing along an optical axis, each incident light beam reflected away from the prism in a direction pointing toward the apex, for producing a plurality of reflected beams that, when incident upon a planar surface substantially normal to the optical axis, form the plurality of light spots arrayed about the optical axis; and
translating the prism along the optical axis between a first position wherein the light spots are separated by a first spacing and a second position wherein the light spots are separated by a second spacing smaller than the first spacing.

37. The method recited in Claim 36, wherein, in performing the translating step, the light spots have a substantially equal size with the prism in the first and the second positions.

38. The method recited in Claim 36, further comprising the steps of analyzing the detected reflected energy and translating the prism in a direction for retaining the light spots on the boundary.

39. The method recited in Claim 33, wherein the adjusting step comprises the steps of:

directing an incident light beam onto each facet of a pyramidal transmissive prism having a plurality of facets meeting at an apex, the apex pointing along an optical axis, each incident light beam refracted within the prism to form a refracted beam in a direction pointing toward the apex, the plurality of refracted beams, when incident upon a planar surface substantially normal to the optical axis, forming the plurality of light spots arrayed about the optical axis; and translating the prism along the optical axis between a first position wherein the light spots are separated by a first spacing and a second position wherein the light spots are separated by a second spacing smaller than the first spacing.

40. The method recited in Claim 39, wherein, in performing the translating step, the light spots have a substantially equal size with the prism in the first and the second positions.

41. The system recited in Claim 39, further comprising analyzing the detected reflected energy and translating the prism in a direction for retaining the light spots on the boundary.

FIG. 1

EP 1 369 079 A1

FIG. 2

FIG. 3

10

FIG. 4

FIG. 5

FIG. 6

FIG. 7

**European Patent**
**Office**

## EUROPEAN SEARCH REPORT

Application Number

EP 03 01 1826

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 315 773 B1 (ZEPKIN NEIL ET AL) 13 November 2001 (2001-11-13) * column 4, line 42 - column 5, line 50; figures 2-4 * | 13,15, 33,35 | A61B3/113 G02B26/08 |
| X | US 6 299 307 B1 (OLTEAN IOAN T ET AL) 9 October 2001 (2001-10-09) * column 14, line 63 - column 15, line 7; figure 10 * * column 16, line 36-39; claim 40 * | 13,33 | |
| X | US 4 439 010 A (DOTY JAMES L) 27 March 1984 (1984-03-27) | 1,2,4-7 | |
| A | * column 4, line 35 - column 5, line 66; figure 5 * | 16,17, 21-25, 36-38 | |
| X | US 5 470 329 A (SUMIYA TOSHIFUMI) 28 November 1995 (1995-11-28) | 1 | |
| A | * column 7, line 4-19; figure 18 * | 16,21,36 | |
| X | US 2001/046038 A1 (MULKENS JOHANNES CATHARINUS H ET AL) 29 November 2001 (2001-11-29) | 8,9,11, 12 | TECHNICAL FIELDS SEARCHED (Int.Cl.7) A61B G02B |
| A | * paragraphs [0046]-[0054]; figures 4-8 * | 19,20, 28-32, 39-41 | |
| X | US 5 675 401 A (RICHTER GERALD ET AL) 7 October 1997 (1997-10-07) | 8 | |
| A | * column 5, line 33 - column 6, line 40 * | 19,28,39 | |

The present search report has been drawn up for all claims

| Place of search | Date of completion of the search | Examiner |
|---|---|---|
| BERLIN | 25 September 2003 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 03 01 1826

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2003

| Patent document cited in search report | | Publication date | Patent family member(s) | | Publication date |
|---|---|---|---|---|---|
| US 6315773 | B1 | 13-11-2001 | US | 5632742 A | 27-05-1997 |
| | | | AT | 232694 T | 15-03-2003 |
| | | | AU | 2382595 A | 16-11-1995 |
| | | | CA | 2188038 A1 | 02-11-1995 |
| | | | DE | 69529681 D1 | 27-03-2003 |
| | | | EP | 0789531 A1 | 20-08-1997 |
| | | | IL | 113354 A | 30-10-1998 |
| | | | JP | 10503940 T | 14-04-1998 |
| | | | WO | 9528879 A1 | 02-11-1995 |
| | | | ZA | 9503144 A | 04-01-1996 |
| US 6299307 | B1 | 09-10-2001 | AU | 1073499 A | 03-05-1999 |
| | | | CA | 2305697 A1 | 22-04-1999 |
| | | | EP | 1026998 A1 | 16-08-2000 |
| | | | JP | 2001519196 T | 23-10-2001 |
| | | | WO | 9918868 A1 | 22-04-1999 |
| US 4439010 | A | 27-03-1984 | NONE | | |
| US 5470329 | A | 28-11-1995 | JP | 2907656 B2 | 21-06-1999 |
| | | | JP | 6078947 A | 22-03-1994 |
| | | | US | 5620437 A | 15-04-1997 |
| US 2001046038 | A1 | 29-11-2001 | EP | 0949541 A2 | 13-10-1999 |
| | | | JP | 2000058441 A | 25-02-2000 |
| | | | TW | 419422 B | 21-01-2001 |
| | | | US | 2002167653 A1 | 14-11-2002 |
| US 5675401 | A | 07-10-1997 | DE | 4421053 A1 | 21-12-1995 |
| | | | DE | 4441947 A1 | 30-05-1996 |
| | | | DE | 59507458 D1 | 27-01-2000 |
| | | | EP | 0687956 A1 | 20-12-1995 |
| | | | JP | 8006175 A | 12-01-1996 |